Europäisches Patentamt

**European Patent Office**

Office européen des brevets

(19)

(11) **EP 1 094 117 A1**

(12) **DEMANDE DE BREVET EUROPEEN**

(43) Date de publication:
**25.04.2001 Bulletin 2001/17**

(51) Int Cl.⁷: **C12P 21/00**, C12H 1/14

(21) Numéro de dépôt: **00402916.1**

(22) Date de dépôt: **20.10.2000**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **22.10.1999 FR 9913231**

(71) Demandeur: **LESAFFRE et Cie**
**F-75001 Paris (FR)**

(72) Inventeur: **Follett, Jean Bernard**
**94140 Alfortville (FR)**

(74) Mandataire: **Koch, Gustave**
**Cabinet PLASSERAUD**
**84, rue d'Amsterdam**
**75440 Paris Cédex 09 (FR)**

(54) **Poudre de mannoprotéines soluble**

(57) L'invention a pour objet une poudre de manno-protéines solubles présentant les caractéristiques suivantes :

- teneur en matière sèche au moins égale à 92%, de préférence au moins égale à 95% ;
- teneur en glucides totaux par rapport à la matière sèche comprise entre 43 et 82%, de préférence entre 43 et 75%, et encore de préférence entre 43 et 70%, encore plus préférentiellement entre 50 et 70% dont au moins 80%, et de préférence au moins 90% sont sous forme d'une chaîne de mannose ;
- teneur en phosphore par rapport à la matière sèche, exprimée en $P_2O_5$, comprise entre 1 et 5,75%, de préférence entre 2,75 % et 5,75 %, et encore de préférence entre 3 et 4,5% ;
- turbidité mesurée par néphélométrie en solution à 1 g/litre dans l'eau à pH 3,5 inférieure ou égale à 6 NTU, de préférence inférieure ou égale à 4 NTU et encore de préférence inférieure ou égale à 3 NTU;
- turbidité mesurée par néphélométrie en solution à 1g/litre dans une solution hydroalcoolique à pH 3,5 et à 12° GL inférieure ou égale à 35 NTU, de préférence inférieure ou égale à 20 NTU et encore de préférence inférieure ou égale à 10 NTU.

Figure unique
PROCEDE D'OBTENTION d'une POUDRE de MANNOPROTEINES SOLUBLES

**Description**

L'objet de l'invention consiste d'une part en une préparation en poudre de mannoprotéines soluble dans l'eau et dans toutes les solutions hydro-alcooliques et d'autre part en une méthode d'obtention d'une poudre desdites mannoprotéines.

Les mannoprotéines sont des molécules complexes correspondant à des mannanes liées à des protéines, dont une des sources les plus intéressantes sont les parois des cellules de levure, dont elles sont un des constituants importants.

Une difficulté non résolue jusqu'à maintenant est d'obtenir industriellement des mannoprotéines en poudre donnant des solutions limpides et ayant de manière reproductible des propriétés rendant intéressant leur emploi dans l'élaboration des denrées et boissons humaines.

L'objet de l'invention est une poudre de mannoprotéines séchées ayant les caractéristiques suivantes :

- teneur en matière sèche au moins égale à 92%, de préférence au moins égale à 95% ;
- de préférence, teneur en protéines (N x 6,25) par rapport à la matière sèche comprise entre 20 et 50%, et encore de préférence entre 29 et 50% ;
- teneur en glucides totaux par rapport à la matière sèche comprise de préférence entre 43 et 82%, de préférence entre 43 et 75%, et encore de préférence entre 43 et 70%, encore plus préférentiellement entre 50 et 70% dont au moins 80% et de préférence au moins 90% sont sous forme d'une chaîne de mannose ;
- de préférence, teneur en phosphore, exprimée en $P_2O_5$, par rapport à la matière sèche comprise entre 1 et 5,75%, de préférence entre 2,75 % et 5,75 %, et encore de préférence entre 3 et 4,5% ;
- la turbidité d'une solution à 1 g/litre dans l'eau de cette poudre de mannoprotéines mesurée par néphélométrie à pH 3,5 est inférieure ou égale à 6 NTU, de préférence inférieure ou égale à 4 NTU et encore de préférence inférieure ou égale à 3 NTU ;
- la turbidité d'une solution hydroalcoolique à 1 g/litre de cette poudre de mannoprotéines mesurée par néphélométrie à pH 3,5 et à 12° GL (°Gay-Lussac correspondant au pourcentage volume/volume d'alcool = éthanol) est inférieure ou égale à 35 NTU, de préférence inférieure ou égale à 20 NTU et encore de préférence inférieure ou égale à 10 NTU.

La turbidité exprimée en NTU (Unité de Turbidité néphélométrique ou encore Unité néphélémétrique de turbidité ou «Nephelometric Turbidity Unit ») est la mesure de la clarté optique d'un liquide. Elle permet d'apprécier la présence de traces de particules colloïdales dans l'eau pure. Cela en fait donc une mesure de choix pour apprécier la solubilité des mannoprotéines dans l'eau ou dans une solution hydroalcoolique. Elle est également très généralement utilisée pour apprécier la limpidité des vins.

La littérature et notamment The Yeasts - Second Edition - Volume 4 : Yeast Organelles p.214-223 edited by A.H. Rose and J.S. Harrison - Academic Press 1991, décrit plusieurs procédés d'obtention (ou de préparation) de mannoprotéines : une préparation par voie physique, une préparation par voie enzymatique. Les procédés décrits ont conduit à différents isolements de mannoprotéines.

Ces mannoprotéines, en général extraites des cellules de levure de l'espèce *Saccharomyces cerevisiae,* ont un ensemble de propriétés intéressantes :

■ des propriétés émulsifiantes,
■ des propriétés de stabilisation des vins contre la casse protéique,
■ des propriétés de stabilisation des vins contre la précipitation tartrique.

La difficulté non résolue est la mise au point d'un procédé permettant d'obtenir à l'échelle industrielle des mannoprotéines ayant les propriétés recherchées, notamment un fort pouvoir protecteur contre la précipitation tartrique des vins et répondant aux critères de solubilité définis ci-dessus. En effet, chez *Saccharomyces cerevisiae* par exemple, les mannoprotéines font partie d'une structure complexe dans laquelle on trouve d'autres glycoprotéines, des polysaccharides, des lipoprotéines et des protéines. Les parois cellulaires représentent environ 30% en poids de la cellule de levure, les mannoprotéines représentant moins de 30% des parois de la cellule de levure.

De manière générale, tous les pourcentages sont exprimés en poids.

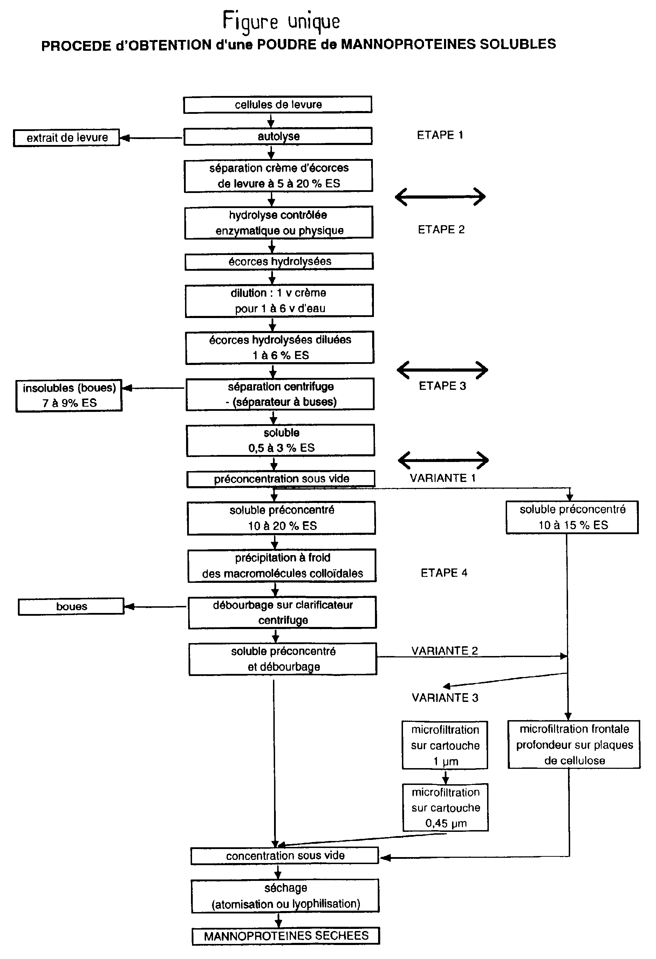

Le procédé d'obtention de mannoprotéines selon l'invention, tel que représenté par exemple sur la figure 1, permet de résoudre cette difficulté.

Ce procédé comprend au moins trois parties ou étapes principales successives. Ce procédé comprend au moins quatre parties, si on distingue dans ces étapes, l'étape obligatoire de la séparation de la fraction solubilisée des parois de cellules de levure après la solubilisation contrôlée et sélective desdites parois :

a) la séparation des parois de cellules de levure (ou écorces de levure) contenant des glucanes et des mannanes du contenu cellulaire, de préférence par autolyse des cellules de levure, de préférence des cellules de *Saccharomyces cerevisiae.* En d'autres termes, les parois de cellules de levure ou écorces de levure sont obtenues par un procédé qui, par une dégradation enzymatique du contenu cellulaire des levures, permet de séparer une fraction insoluble contenant principalement les parois des cellules de levure ;

b) la solubilisation sélective des parois de cellules de levure issues de l'étape ci-dessus par un procédé d'hydrolyse contrôlée physique ou enzymatique permettant de récupérer une fraction solubilisée riche en mannoprotéines ;

c) la séparation des fractions solubilisées et insolubles pour ne conserver que la fraction solubilisée riche en mannoprotéines ;

d) la purification de ladite fraction solubilisée en vue de l'obtention de mannoprotéines solubles, ayant les propriétés recherchées ;

La fraction solubilisée purifiée est ensuite concentrée et éventuellement séchée.

Ces trois parties ou étapes principales du procédé selon l'invention : a), b) et d), se caractérisent par les mesures suivantes :

### a) séparation des parois des cellules de levure (ou écorces de levure) du contenu cellulaire des levures

La première étape du procédé selon l'invention consiste à solubiliser sélectivement le contenu cellulaire des cellules de levures, de manière à obtenir une fraction insoluble correspondant essentiellement aux parois cellulaires ou écorces. Le moyen préféré pour opérer cette solubilisation sélective du contenu cellulaire, c'est de recourir à une autolyse des cellules de levure selon les procédés classiques de fabrication des extraits de levure.

L'autolyse des cellules de levure est définie de manière générale comme la solubilisation ou hydrolyse du contenu cellulaire des cellules de levure par les enzymes internes aux cellules de levure, avec ajout éventuellement à la suspension de cellules en autolyse de protéases, comme par exemple la papaïne. L'autolyse de cellules de levure est notamment décrite pages 370 à 377 dans l'ouvrage de référence "Yeast Technology", 2nd edition, 1991, G. Reed and T.W. Nagodawithana, published by Van Nostrand Reinhold, New York, ISBN 0-442-31892-8.

Le procédé d'autolyse d'une crème de levure (suspension de cellules de levure dans de l'eau), de préférence d'une crème de levures de boulangerie *Saccharomyces cerevisiae,* est conduit de façon à pouvoir séparer des parois de cellules de levure, aptes à être utilisées comme matière première pour la production de mannoprotéines.

On obtient après séparation de la fraction solubilisée appelée généralement extrait de levure ou autolysat de levure, des parois de cellules de levure ou écorces de levure aptes à être utilisées pour la production de mannoprotéines notamment lorsque l'autolyse de la crème de levure est conduite de manière à solubiliser entre environ 50% et environ 73% des matières sèches cellulaires, de préférence entre 60 et 70% desdites matières sèches cellulaires. En d'autres termes, la première étape du procédé selon l'invention est une étape d'obtention d'une suspension de parois de cellules de levure ayant des caractéristiques identiques ou similaires à celles d'une suspension de parois de cellules de levure obtenus après une autolyse desdites cellules de levure solubilisant environ 50% à environ 73% des matières sèches totales des cellules de levure, de préférence 50 à 70% et encore de préférence 60 à 70% desdites matières sèches des cellules de levure.

Ce taux de solubilisation (T.S.) :

$$T.S. = \frac{\text{quantité de matière sèche soluble après hydrolyse}}{\text{quantité de matière sèche de départ}} = \frac{S}{D}$$

est généralement estimé par la formule approximative suivante :

$$T.S. = \frac{ESs \times (100 - ESd)}{(100 - ESs) \times ESd}$$

où ESd = Extrait sec en % de l'ensemble en suspension ou extrait sec total de départ.

ESs = Extrait sec en % de la solution obtenue après hydrolyse et séparation de l'insoluble. Etant précisé que l'on considère dans la présente demande que les expressions « extrait sec » et « matières sèches » ou « ES » ou « matière sèche » sont synonymes.

Les matières sèches sont mesurées de préférence à l'étuve à environ 105°C, l'échantillon dont les matières sèches sont mesurées étant laissé, comme usuel selon les bonnes pratiques de laboratoire, dans l'étuve, jusqu'à ce que le poids après dessiccation soit constant.

Cette formule est basée sur l'hypothèse que la quantité d'eau de la suspension de départ ayant une concentration ESd en matière sèche et celle de la solution résultat de la solubilisation et ayant une concentration ESs en matière sèche, sont constantes. Cette hypothèse pose problème du fait des riques d'évaporation, et du fait aussi que l'hydrolyse mobilise, par définition, une certaine quantité d'eau, en général négligeable. Si la quantité d'eau liée par l'hydrolyse est négligeable par rapport au poids d'eau total, un moyen pour ne pas avoir à tenir compte de l'évaporation lors de l'hydrolyse est de mesurer ESd sur une suspension homogène réalisée après l'hydrolyse et, avant séparation des diverses fractions (ESd = Extrait sec total de l'ensemble). Si les artefacts ne sont pas négligeables, des bilans doivent être faits pour corriger cette formule.

La fraction insoluble contenant essentiellement les parois de cellules de levure est séparée de l'extrait de levure solubilisé par l'autolyse par des moyens classiques, en général par centrifugation, de préférence après une pasteurisation de l'ensemble. Cette fraction insoluble correspondant à une suspension de parois de cellules de levure fera de préférence l'objet d'un lavage important, de manière à bien éliminer de la suspension de parois cellulaires ou écorces de levure les fractions solubilisées du contenu cellulaire qui pourraient être retenues.

De préférence, la suspension issue de l'autolyse contenant la fraction insoluble, obtenue après séparation de la fraction solubilisée par l'autolyse, et qui sera soumise à la seconde étape de solubilisation des mannoprotéines par hydrolyse contrôlée aura la composition suivante :

- teneur en matière sèche comprise entre 5 et 20%, de préférence entre 10 et 15% et encore de préférence entre 12 et 13%,
- teneur en azote par rapport à la matière sèche comprise entre 1,5 et 3,5%, de préférence entre 1,5 et 3%, et encore de préférence entre 1,5 et 2,5%, et encore plus préférentiellement entre 1,5 et 2%,
- teneur en glucides totaux par rapport à la matière sèche comprise entre 40 et 60%, parmi lesquels entre 30 et 40 % de ces glucides sont sous forme de mannanes,
- teneur en lipides par rapport à la matière sèche comprise entre 20 et 30%,
- teneur en matières minérales par rapport à la matière sèche comprise entre 5 et 10%,
- teneur en phosphore, exprimée en $P_2O_5$, par rapport à la matière sèche comprise entre 2 et 5%, de préférence entre 1,8 et 4%, encore de préférence entre 1,8 et 2,3%
- pH compris entre 5 et 7, de préférence entre 6 et 7.

L'homme de l'art connaît bien les méthodes adaptées à l'analyse des levures et des fractions de levures, de manière à obtenir des résultats analytiques aussi exacts que possible. L'inventeur a employé plus particulièrement les méthodes dont les principes généraux sont décrits dans la présente description ci-dessus et ci-dessous .

La teneur en matière sèche ou extrait sec (mesure à l'étuve comme indiqué ci-dessus), l'azote (azote Kjedhal), le $P_2O_5$, sont mesurés selon les méthodes conventionnelles classiques.

La teneur en protéines correspond aux protéines conventionnelles (N x 6,25).

Les matières minérales sont mesurées par incinération à 800°C pendant 18 heures.

Les lipides sont dosés par une méthode gravimétrique de type DRAPRON (hydrolyse par HCl concentré et chaud, récupération soigneuse du résidu après lavage, extraction au Soxhlet à chaud sous reflux avec éther de pétrole).

La teneur en glucides totaux est exprimée sur la base d'un équivalent mannose et dosée selon une méthode classique de coloration à l'anthrone après hydrolyse acide.

La teneur en glucanes ou mannanes est estimée par le dosage spécifique du glucose ou du mannose après hydrolyse des molécules de glucanes ou mannanes selon les procédés classiques d'hydrolyse des glucides afin de les doser. Toutes les valeurs données sont donc indiquées à partir d'un dosage d'un équivalent glucose ou mannose.

Le mannose peut être notamment dosé des deux manières suivantes :

1. Dosage par voie enzymatique.

Après hydrolyse ménagée à chaud des parois de cellules de levure (écorces de levure) avec de l'acide chlorhydrique (2N), ce qui permet d'hydrolyser les polymères de glucanes et de mannanes, le mannose est dosé spécifiquement par voie enzymatique selon les réactions suivantes :

■ Mannose + ATP --> Mannose-6-P + ADP sous l'action d'une hexokinase (HK).
■ Mannose-6-P --> Fructose-6-P sous l'action d'une phosphomannose isomérase (PMI) spécifique.
■ Fructose 6-P --> Glucose-6-P sous l'action d'une phosphoglucose isomérase (PGI).
■ Le Glucose 6-P réagit à son tour avec le NADP, NADP qui est transformé en NADPH.
■ Le NADPH libéré est dosé par spectrophotométrie à 340 nm.

Il est également possible de doser spécifiquement le glucose par voie enzymatique et de calculer ainsi le pourcentage de résidus mannanes par rapport aux glucides (Rapport teneur en mannose sur teneur en mannose et glucose).

Les enzymes nécessaires à ces dosages enzymatiques spécifiques du glucose et du mannose peuvent être achetées sous forme de kits de dosage par exemple à la société Boehringer Mannheim GmbH, maintenant Roche Diagnostic.

2. <u>Dosage par la méthode à l'anthrone après extraction et purification de la fraction mannanes.</u>

Les parois de cellules de levure (ou écorces de levure) sont traitées dans un premier temps à l'acide trichloracétique, ce qui permet d'éliminer la fraction soluble contenant éventuellement le tréhalose.

La partie insoluble est traitée ensuite au $Na_2CO_3$ (carbonate de sodium) pour éliminer les glucanes et éventuellement le glycogène acide qui restent dans la partie insoluble.

La partie solubilisée contenant les mannanes et éventuellement le glycogène alcalin est traitée à la soude et au sulfate de cuivre, ce qui précipite les mannanes qui sont récupérés par simple filtration.

Ce précipité est re-dissous et hydrolysé complètement avec de l'acide sulfurique et le dosage des mannanes est effectué sur la solution obtenue par la méthode colorimétrique à l'anthrone de dosage des sucres réducteurs en utilisant une gamme étalon mannose.

De manière générale, un bilan a peu près exact ne peut être obtenu qu'en combinant ces différentes méthodes. Toutes les mesures de glucides par hydrolyse sont en théorie par excès car l'hydrolyse lie une partie de l'eau, par contre cette hydrolyse peut être incomplète.

**b) <u>hydrolyse contrôlée des parois de cellules de levure</u>**

La solubilisation spécifique ou sélective des mannoprotéines contenues dans les parois de cellules de levure issus de la première étape ou première partie du procédé est obtenue par hydrolyse contrôlée soit par voie physico-chimique, soit par voie enzymatique.

Cette solubilisation est appelée improprement hydrolyse des parois de cellules de levure. Dans ces parois ou écorces insolubles, les mannoprotéines sont emprisonnées dans un réseau notamment formé par des glucanes. Il est nécessaire de désagréger ce réseau soit par voie physicochimique (par exemple en solution aqueuse, sous l'action de la température) soit par voie enzymatique (par exemple sous l'action de glucanases).

Cette hydrolyse ou solubilisation doit être menée de façon contrôlée et sélective de manière :

- d'une part à libérer un maximum de mannoprotéines présentes ;
- d'autre part à libérer un minimum de macromolécules colloïdales complexes qui vont former des troubles, et avoir rapidement un effet colmatant dans toutes les opérations d'épuration par filtration.

En effet, le procédé selon l'invention réside dans une sélection d'étapes permettant d'obtenir une fraction solubilisée où la quasi-totalité des macromolécules présentes sont des mannoprotéines, avec un minimum d'autres macromolécules susceptibles de former des troubles gênants. L'avantage dans le procédé selon l'invention de séparer d'abord une fraction insoluble contenant essentiellement les parois des cellules de levures est d'éliminer dès ce stade toutes les autres macromolécules appartenant au contenu cellulaire et susceptibles de se solubiliser en même temps que les mannoprotéines. La solubilisation des parois cellulaires doit être sélective, c'est-à-dire contrôlée de manière à être arrêtée, lorsque des macromolécules colloïdales en qualité ou en quantité rendant pratiquement impossible leur élimination risquent d'être solubilisées avec les mannoprotéines. En effet, il ne suffit pas de solubiliser les mannoprotéines, le problème le plus délicat est d'obtenir une fraction purifiée sans hydrocolloïdes provoquant des troubles en solution. L'hydrolyse doit être contrôlée de manière à permettre la séparation d'une fraction solubilisée contenant les mannoprotéines solubilisées avec uniquement des macromolécules colloïdales pouvant être éliminées lors du traitement de purification. Si l'hydrolyse est trop importante, une quantité supérieure de mannanes peut être extraite, mais l'extrait devient pratiquement impossible à purifier, quel que soit l'ensemble des techniques mises en oeuvre. En conséquence, cet extrait devient notamment inutilisable en vinification, car il va créer un trouble inacceptable dans le vin.

L'hydrolyse contrôlée des parois de cellules de levure se fait de préférence à l'aide de la chaleur et est conduite de manière à ce que le taux de solubilisation de la fraction solubilisée, calculé selon la formule :

$$T.S = \frac{ESs \times (100 - ESd)}{(100 - ESs) \times ESd}$$

avec ESd mesuré sur une suspension homogène de l'ensemble réalisée après hydrolyse et avant séparation des diverses fractions, soit compris entre 15 et 26% de la matière sèche mises en oeuvre et de préférence entre 18 et 25%, et encore de préférence entre 17 et 20%.

De préférence, la solubilisation des mannoprotéines est opérée par hydrolyse thermique à une valeur de pH comprise entre 6 et 7.

L'hydrolyse thermique contrôlée des parois de cellules de levure est menée de préférence entre pH 6 et pH 7, de préférence entre 95 et 100°C, et de préférence pendant une durée comprise entre 15 et 30 heures et encore de préférence entre 17 et 25 heures, et encore de préférence entre 17 et 22 heures.

De manière générale, la sélection du taux de solubilisation dépend de la fraction de parois insolubles de cellules de levure mise en oeuvre, du procédé de solubilisation et des résultats des mesures de turbidité faites après séparation de la fraction solubilisée. Il peut dépendre également de l'application recherchée.

Le principal critère est le suivant et il est valable quel que soit le procédé de solubilisation :

l'étape d'hydrolyse contrôlée des parois de cellules de levure est conduite de manière à ce que la fraction solubilisée obtenue après l'étape d'hydrolyse et après l'étape de séparation de la fraction insoluble, ait une valeur de turbidité de moins de 30 NTU, de préférence de moins de 20 NTU, et encore de préférence de moins de 15 NTU et encore plus préférentiellement de moins de 10 NTU lorsqu'elle est mesurée par néphélométrie dans une solution aqueuse diluée contenant 1% de matières sèches, à une valeur de pH comprise entre 5 et 7, de préférence entre 6 et 7.

**c) séparation de la fraction insoluble des écorces hydrolysées pour ne conserver que la fraction solubilisée**

Après l'étape d'hydrolyse contrôlée, la suspension de parois de cellules de levure hydrolysées (ou crème d'écorces de levure hydrolysées) est diluée par 2 à 6 volumes d'eau (de préférence à une température comprise entre 1°C et 25°C) jusqu'à une concentration comprise entre 1 et 6% de matière sèche, de préférence entre 3 et 5% de matière sèche, avant de procéder à la séparation de la fraction solubilisée riche en mannoprotéines et de la fraction insoluble. Cette dilution a pour objectif de rendre plus efficace la séparation de l'insoluble, en particulier lorsque cette séparation est effectuée par centrifugation.

Les matières solubilisées riches en mannoprotéines sont alors séparées des matières demeurant insolubles.

De préférence, la fraction solubilisée est séparée par centrifugation de la partie insoluble.

Dans les conditions décrites ci-dessus, la séparation centrifuge sur séparateur à buses donne un surnageant d'indice turbidimétrique inférieur à 30 NTU pour une teneur en matière sèche d'environ 1,0%, de préférence moins de 20 NTU et encore de préférence moins de 15 NTU.

Ces opérations conduisent à une solution après séparation (surnageant de centrifugation si la séparation est faite par centrifugation) ayant les caractéristiques suivantes :

- teneur en matière sèche comprise entre 0,5 et 3%, de préférence entre 0,5% et 1,5% ;
- teneur en protéines (N x 6,25) par rapport à la matière sèche comprise entre 15 et 55%, de préférence entre 20 et 50% ;
- teneur en glucides totaux par rapport à la matière sèche comprise entre 40 et 80 %, de préférence entre 40 et 75%, et encore de préférence entre 50 et 67% dont au moins 75% de mannanes et de préférence au moins 80% de mannanes, et encore de préférence au moins 85% de mannanes ;
- teneur en phosphore, exprimée en $P_2O_5$ par rapport à la matière sèche comprise entre 1 et 6,00%, de préférence entre 2,75 et 6,00%, et encore de préférence entre 2,75 et 4,5%;
- pH compris entre 6 et 7.

Après séparation de la fraction insoluble, la fraction solubilisée, lorsqu'elle est diluée ou ajustée à une concentration de 1% de matière sèche dans l'eau, présente une turbidité néphélométrique mesurée à une valeur de pH comprise entre 5 et 7 de moins de 30 NTU, de préférence moins de 20 NTU, et encore de préférence moins de 15 NTU et encore plus préférentiellement moins de 10 NTU.

**d) Purification des mannoprotéines**

La purification des mannoprotéines est pratiquement impossible à réaliser quand l'hydrolyse des parois de cellules de levure est menée sans ménagement et conduit à la libération de trop d'hydrocolloïdes. Par contre, si l'hydrolyse est menée à partir des parois de cellules de levure obtenus selon la partie 1 du procédé selon l'invention, de façon ménagée (c'est-à-dire contrôlée et sélective) selon la partie 2 du procédé selon l'invention, la purification peut être menée de manière très simple.

Lorsque l'hydrolyse contrôlée des parois de cellules de levures a été opérée par voie enzymatique de manière à ce que la fraction solubilisée après l'étape de séparation ait une valeur de turbidité de moins de 30 NTU, de préférence de moins de 20 NTU, lorsqu'elle est mesurée par néphélométrie dans une solution aqueuse diluée contenant 1% de matière sèche, à pH entre 5 et 7, une ultrafiltration/diafiltration est nécessaire pour éliminer les molécules de poids moléculaire inférieur à 10000 daltons libérées par l'action de la glucanase. En effet, de manière générale, les mannoprotéines selon l'invention sont constituées essentiellement de molécules ayant un poids moléculaire supérieur à 10000 daltons. Les ultrafiltrats/diafiltrats sont éliminés et le retentat qui est trouble peut être purifié par une réfrigération à 4°C pendant 24 heures suivie d'une clarification, cette clarification étant suivie si nécessaire ou remplacée par une microfiltration en profondeur, ces étapes de purification étant les mêmes que celles décrites ci-après.

Lorsque l'hydrolyse contrôlée a été conduite par voie physicochimique, par exemple par voie thermique comme indiqué ci-dessus, pour obtenir une solution de mannoprotéines ayant les caractéristiques de turbidité rappelées dans les paragraphes ci-dessus, ladite solution est soumise aux différentes étapes d'extraction-purification suivantes :

- préconcentration de la fraction solubilisée et séparée, de préférence par évaporation sous vide, jusqu'à des valeurs comprises entre 10 et 20% de matière sèche, de préférence entre 13 et 17% de matière sèche;
- réfrigération de la fraction préconcentrée et maintien à une température comprise entre 0 et 6°C jusqu'à ce que sa turbidité n'augmente plus. Ce traitement au froid se fait de préférence pendant au moins 24 heures ;
- séparation des macromolécules colloïdales précipitées au froid par clarification centrifuge. La clarification centrifuge est réalisée de préférence à au moins 10 000 g sur séparateur auto-débourbeur ;

cette purification étant suivie des étapes complémentaires suivantes :

- concentration, par exemple par évaporation sous vide, de l'extrait clarifié ;
- éventuellement, séchage (par exemple par atomisation en conditions ménageantes ou par lyophilisation).

Au cours de l'opération de préconcentration, la turbidité de la solution augmente de façon importante. Elle est multipliée par un facteur 5 à 10 à matière sèche égale. L'étape de clarification à froid permet de diviser cette turbidité par un facteur 3.

Des variantes à ce procédé peuvent être introduites après l'étape de préconcentration ou après la précipitation par le froid et la clarification centrifuge et avant la concentration. Ces variantes consistent en une microfiltration frontale en profondeur permettant d'éliminer tous les composés de nature colloïdale, par exemple de type lipoprotéines ou glycoprotéines (autres que les mannoprotéines), qui peuvent subsister et apporter un trouble à l'extrait de mannoprotéines, afin d'obtenir un produit final soluble.

Plusieurs techniques de microfiltration en profondeur sont envisageables et notamment par exemple :

**1.** La filtration sur plaques de cellulose.

Cette technique permet d'abaisser l'indice turbidimétrique de la solution concentrée de mannoprotéines à 5 NTU ou moins de 5 NTU en solution normalisée à 1% de matière sèche avec un seuil de rétention du filtre en profondeur de 10μm. (valeur du pH comprise entre 6 et 7).

L'extrait de mannoprotéines est envoyé au moyen d'une pompe volumétrique dans un filtre en acier inoxydable, stérilisable, pourvu de plateaux entre lesquels sont positionnées les plaques filtrantes - la filtration est effectuée sous légère pression (0,5 bar) - l'élévation de pression révèle un colmatage des média de filtration.

Les plaques filtrantes sont en cellulose - elles retiennent non seulement les particules dont la taille est supérieure à leur seuil d'arrêt (1 à 20 μm, selon le modèle utilisé) mais aussi les troubles colloïdaux par effet d'adsorption dans la masse des fibres cellulosiques.

L'emploi de plaques de cellulose imprégnées de charbon actif est également intéressant pour éliminer d'éventuels résidus de petits poids moléculaires aromatiques et colorés, normalement éliminés lors des étapes précédentes.

**2.** La filtration sur cartouches.

La filtration sur cartouches dont le seuil d'arrêt est supérieur ou égal à 1 μm est insuffisante : seules les particules de taille supérieure à ce seuil sont arrêtées ; l'effet d'adsorption des troubles colloïdaux est moindre, il est partiel et non durable.

Pour éviter cet inconvénient et obtenir un jus parfaitement clarifié, équivalent au filtrat obtenu sur plaque cellulose, il est nécessaire de repasser le filtrat précédent sur une deuxième cartouche de 0,45 μm.

Le procédé selon l'invention conduit à l'obtention de poudres de mannoprotéines sèches ayant les caractéristiques suivantes :

- teneur en matière sèche au moins égales à 92%, de préférence au moins égales à 95% ;
- de préférence, teneur en protéines par rapport à la matière sèche comprise entre 20 et 50%, de préférence entre 29 et 50% ;
- teneur en glucides totaux par rapport à la matière sèche comprise entre 43 et 82%, de préférence entre 43 et 70%, et encore de préférence entre 50 et 65%, dont au moins 80% et de préférence au moins 90%, sont sous forme d'une chaîne de mannose ;

- teneur en phosphore, exprimée en $P_2O_5$, par rapport à la matière sèche comprise entre 1 et 5,75%, de préférence entre 2,75 et 5,75%, et encore de préférence entre 2,75 et 4,5%.

Les préparations en poudre de mannoprotéines obtenues selon l'invention se caractérisent par le fait qu'en solution à 1 g/l dans l'eau elles donnent une turbidité mesurée par néphélométrie à pH 3,5 inférieure ou égale à 6 NTU, de préférence inférieure ou égale à 4 NTU et encore de préférence inférieure ou égale à 3 NTU et qu'en solution à 1 g/litre dans une solution hydroalcoolique à pH 3,5 et à 12° GL, elles donnent une turbidité mesurée par néphélométrie inférieure ou égale à 35 NTU, de préférence inférieure ou égale à 20 NTU et encore de préférence inférieure ou égale à 10 NTU.

Toutes les mesures de turbidité sont réalisées à température ambiante (20°C) avec un Turbidimètre de marque Hach Ratio®, Modèle 18900, vendu par la société Hach, régulièrement étalonné et possédant 3 plages de mesure : 0-2 NTU, 2-20 NTU, 20-200 NTU. Cette mesure dépend du pH mais est indépendante de la température si celle-ci est comprise entre 15°C et 25°C.

L'invention a également pour objet les poudres de mannoprotéines solubles obtenues par un procédé selon l'invention, décrit ci-dessus.

## EXEMPLE 1 :

### a) Préparation des parois de cellules de levure ou écorces de levure.

Une crème de cellules de levure (suspension de cellules de levure dans l'eau) de l'espèce *Saccharomyces cerevisiae* ayant entre 12 et 18% de matière sèche (d'extrait sec) est autolysée à l'aide des enzymes internes des dites levures selon les procédés classiques, avec ajout ou non de protéases d'origine externe, telle que par exemple la papaïne. La crème de levure après autolyse est pasteurisée et séparée en deux fractions :

- l'extrait solubilisé de levures ou extrait de levure ou autolysat de levure correspondant à la digestion ou hydrolyse du contenu cellulaire des levures par ses propres enzymes et les protéases éventuellement ajoutées ;
- une fraction insoluble contenant essentiellement la paroi et la membrane des cellules de levure, cette fraction représente environ 30 à 50% de la matière sèche des levures mises en oeuvre.

Cette première phase est conduite de manière à solubiliser entre 50 et 70% des cellules de levure, de préférence environ 60% des cellules de levure. La fraction insoluble contenant essentiellement les parois de cellules de levure est obtenue après séparation sous forme d'une suspension dans l'eau, cette suspension de parois cellulaires ayant une teneur en matière sèche comprise entre 10 et 15%, de préférence environ 12,5% de matière sèche. Ces matières sèches de parois cellulaires ont la composition suivante

- teneur en azote par rapport à la matière sèche comprise entre 1,5 et 3%, dans cet exemple environ 2,5% ;
- teneur en glucides totaux par rapport à la matière sèche comprise entre 40 et 60%, ces glucides contenant entre 30 et 40% de mannanes ;
- teneur en lipides par rapport à la matière sèche comprise entre 20 et 30% ;
- teneur en matières minérales par rapport à la matière sèche comprise entre 5 et 10% ;
- teneur en phosphore, exprimée en $P_2O_5$, par rapport à la matière sèche comprise entre 2 et 3%.

### b) Hydrolyse contrôlée des parois de cellules de levure issues de l'autolyse des levures.

L'hydrolyse contrôlée des parois de cellules de levure est menée entre pH 6 et 7, à quasi ébullition (entre 98 et 100°C) pendant 25 heures. Le taux de solubilisation de la matière sèche est de 24%.

### c) Séparation des fractions solubilisées et insolubles issues de l'étape d'hydrolyse contrôlée.

La fraction solubilisée (contenant les mannoprotéines) est récupérée, après dilution de la suspension hydrolysée par 2 à 3 fois son volume d'eau froide (à une température comprise entre 10 et 20°C), en écartant la fraction insoluble par centrifugation sur séparateur à buses (1 seul étage).

Le taux de récupération de la fraction solubilisée limpide est d'environ 65%, le taux de récupération étant défini ici comme le rapport exprimé en pourcentage de la masse de la fraction séparée et récupérée sur la masse totale avant séparation.

Dans ces conditions, la séparation donne un surnageant (fraction solubilisée) d'indice turbidimétrique égal à 10 NTU pour une teneur en matière sèche d'environ 1%, la solution (fraction solubilisée) après séparation ayant les caractéristiques suivantes :

- teneur en matière sèche comprise entre 0,5 et 3%, de préférence entre 0,5% et 1,5% ;
- teneur en protéines (N x 6,25) par rapport à la matière sèche comprise entre 15 et 50%, de préférence entre 30 et 50% ;
- teneur en glucides totaux par rapport à la matière sèche comprise entre 40 et 80%, de préférence entre 40 et 67%, dont au moins 80% de mannane, de préférence au moins 85% et encore de préférence au moins 90% de mannane ;
- teneur en phosphore, exprimée en $P_2O_5$, par rapport à la matière sèche comprise entre 2,75 et 4,5%.

## d) Purification de la fraction solubilisée obtenue à l'issue de l'étape de séparation (étape précédente).

La fraction solubilisée et séparée est préconcentrée par évaporation sous vide du surnageant de séparation. La solution impure de mannoprotéines est amenée à une teneur en matière sèche comprise entre 10 et 15%.

La fraction préconcentrée est réfrigérée puis maintenue à 4°C pendant au moins 24 heures.

Les macromolécules colloïdales précipitées au froid sont séparées par clarification centrifuge réalisée à 10 000 g minimum sur séparateur autodébourbeur de type WESTFALIA ®. Le taux de récupération de la fraction solubilisée épurée est de 95%.

Après concentration en boule (évaporateur sous vide à double enveloppe) jusqu'à 30% de matière sèche et séchage ménageant par atomisation, on obtient une poudre fine, de couleur crème, peu odorante, soluble dans l'eau.

Cette poudre de mannoprotéines a une turbidité en solution aqueuse ou dans le vin mesurée par néphélométrie à la concentration de 1 g/l :

- dans une solution aqueuse à pH 3,5 : 2,6 NTU
- dans une solution hydroalcoolique à 12°GL et à pH 3,5: 8,8 NTU
- dans différents vins blancs : comprise entre 10 et 12 NTU.
- La turbidité de ces mannoprotéines à pH neutre est de 1 NTU (NTU eau pure : 0,15) à 1 g/litre.

La turbidité des poudres de mannoprotéines a été déterminée en fonction de la concentration en mannoprotéines :

- d'une part, dans de l'eau distillée à pH compris entre 6 et 7 et,
- d'autre part, dans de l'eau à pH 3,5,

Les résultats sont donnés dans le tableau I ci dessous.

TABLEAU I

| concentration des mannoprotéines (en g/1) | 0,1 | 0,2 | 0,5 | 1 | 2 | 5 | 10 |
|---|---|---|---|---|---|---|---|
| turbidité en NTU dans l'eau distillée à pH entre 6 et 7 | 0,4 | 0,6 | 0,7 | 1 | 2 | 4 | 8,5 |
| turbidité en NTU dans l'eau à pH 3,5 | 1,3 | 1,9 | 2,2 | 2,6 | 5,3 | 11,8 | 22 |

Les mannoprotéines sèches obtenues ont les caractéristiques suivantes :

- teneur en matière sèches : 96% ;
- teneur en protéines (N x 6,25) par rapport à la matière sèche : 43% ;
- teneur en glucides totaux par rapport à la matière sèche : 50% dont 94% de mannanes ;
- teneur en phosphore, exprimée en $P_2O_5$, par rapport à la matière sèche : 3,1% ;
- teneur en azote par rapport à la matière sèche : 6,9% ;
- teneur en matières minérales par rapport à la matière sèche : 6,7% ;
- teneur en glucanes par rapport à la matière sèche : 3%.

L'efficacité des poudres de mannoprotéines ainsi obtenues pour la stabilisation tartrique des vins a été testée sur plusieurs vins différents, rouges ou blancs, provenant de différentes origines et de différents cépages.

Les poudres de mannoprotéines sont apportées à chaque vin en solution concentrée à la dose de 0,2 g de poudre de mannoprotéines par litre de vin, celui-ci est ensuite de préférence filtré sur membrane à 0,45 micromètre.

Ces essais ont montré que les mannoprotéines ainsi obtenues, ajoutées à 0,2 g/litre à différents vins :

- n'interfèrent pas avec la perception gustative des vins (test triangulaire d'évaluation sensorielle),
- affectent peu leur turbidité,
- dans le cadre d'essais de stabulation des vins à -4°C, retardent selon les vins de 3 jours à au moins 17 jours l'apparition des cristaux de bitartrate de potassium.

Cet exemple d'application industrielle des poudres de mannoprotéines est un exemple parmi d'autres, il n'a pas de caractère limitatif.

**EXEMPLE 2 :**

**a) Préparation des parois de cellules de levures**

Une crème de cellules de levure (Saccharomyces cerevisiae) ayant environ 15% de matières sèches est autolysée selon les procédés classiques, avec ajout de protéases au début de l'autolyse.

Le taux de solubilisation à la fin de l'autolyse des cellules de levure est compris entre 65 et 70%, lors de la pasteurisation de l'ensemble de la suspension, pasteurisation qui correspond à la fin du traitement d'autolyse des cellules de levures entières.

La séparation de la fraction solubilisée correspondant à l'extrait de levure valorisé en alimentation humaine et de la fraction insoluble contenant les parois cellulaires est opérée par centrifugations successives, avec remise en suspension des parois de cellule de levure dans l'eau, de manière à obtenir des parois cellulaires ou écorces de levure bien lavées, c'est-à-dire retenant un minimum de fractions cellulaires dissoutes.

Lesdites parois de cellules de levure ont la composition suivante, rapportée à leur matière sèche :

teneur en azote sur matières sèches : 1,8%
teneur en $P_2O_5$ sur matières sèches : 1,95%

**b) Hydrolyse contrôlée des parois de cellules de levure issues de l'étape ci-dessus**

L'hydrolyse contrôlée des parois cellulaires issues de l'étape ci-dessus est menée entre pH 6 et 7, à quasi ébullition, c'est-à-dire pratiquement 100°C pendant 19 heures 30 minutes. Le taux de solubilisation est de 18%.

**c) Séparation des fractions solubilisées et insolubles issues de l'hydrolyse contrôlée**

La fraction solubilisée contenant les mannoprotéines est récupérée comme dans l'étape correspondante de l'exemple 1 ci-dessus. Cette fraction solubilisée a la composition approximative suivante, ramenée à ses matières sèches :

protéines sur matières sèches : 26%
matières minérales sur matières sèches : 8%
glucides sur matières sèches : 66%, dont au moins 90% de mannanes.

**d) Purification de la fraction solubilisée de mannoprotéines obtenue à l'issue de l'étape précédente**

Cette purification est menée comme dans l'étape correspondante de l'exemple 1, avec une préconcentration, une réfrigération à 4°C et une clarification centrifuge à 15000g.

Cette purification est suivie d'une nouvelle concentration et d'un séchage par atomisation.

Le produit obtenu a les caractéristiques suivantes :

teneur en matières sèches : 96%
teneur en N sur matière sèche : 4,1%, soit teneur en protéines sur matières sèches 4,1 x 6,25 = 25,6%
teneur en $P_2O_5$ sur matière sèche : 4,2%
teneur en matières minérales sur matière sèche : 8,2%
teneur en mannanes sur matière sèche : 64%
teneur en glucanes sur matière sèche : 1,2%

Cette poudre de mannoprotéines a une turbidité en solution à la concentration de 1 g/l

dans une solution aqueuse à pH neutre : 1 NTU

dans une solution aqueuse à pH 3,5 : 3 NTU
dans une solution hydroalcoolique à 12°GL et à pH 3,5 : 6 NTU

**Revendications**

1. Poudre de mannoprotéines solubles caractérisée en ce :

   - qu'elle a une teneur en matière sèche au moins égale à 92%, de préférence au moins égale à 95% ;
   - qu'elle a une teneur en glucides totaux par rapport à la matière sèche comprise entre 43 et 82%, de préférence entre 43 et 70%, et encore de préférence entre 50 et 70% dont au moins 80%, et de préférence au moins 90% sont sous forme d'une chaîne de mannose ;
   - qu'elle a une teneur en phosphore, exprimée en $P_2O_5$, par rapport à la matière sèche comprise entre 1% et 5,75%, de préférence entre 2,75 et 5,75%, et encore de préférence entre 3 et 4,5% ;
   - qu'une mesure de turbidité d'une solution à 1 g/litre de ladite poudre de mannoprotéines dans de l'eau réalisée à pH 3,5 par néphélométrie donne une valeur inférieure ou égale à 6 NTU, de préférence inférieure ou égale à 4 NTU et encore de préférence inférieure ou égale à 3 NTU ;
   - qu'une mesure de turbidité d'une solution à 1 g/litre de ladite poudre de mannoprotéines dans une solution hydroalcoolique réalisée à pH 3,5 et à 12°GL par néphélométrie donne une valeur inférieure ou égale à 35 NTU, de préférence inférieure ou égale à 20 NTU et encore de préférence inférieure ou égale à 10 NTU.

2. Poudre de mannoprotéines solubles selon la revendication 1 caractérisée en ce :

   - qu'elle a une teneur en glucides totaux par rapport à la matière sèche comprise entre 43 et 75%, de préférence entre 43 et 70%, et encore de préférence entre 50 et 70%, dont au moins 80%, et de préférence au moins 90% sont sous forme d'une chaîne de mannose ;
   - qu'elle a une teneur en protéines par rapport à la matière sèche comprise entre 20 et 50%, de préférence entre 29 et 50%.

3. Procédé d'obtention de mannoprotéines solubles caractérisé en ce qu'il comprend au moins les trois étapes successives principales suivantes a), b), et d) :

   a) une étape d'obtention d'une suspension de parois de cellules de levure ayant des caractéristiques identiques ou similaires à celles d'une suspension de parois de cellules de levure obtenus après une autolyse desdites cellules de levure solubilisant entre 50 et 73% des matières sèches des cellules de levure, de préférence entre 50 et 70%
   b) une étape d'hydrolyse contrôlée desdites parois de cellules de levure de manière à permettre d'une part la solubilisation sélective des mannoprotéines à cette étape et d'autre part la séparation à une étape ultérieure d'une fraction solubilisée contenant les mannoprotéines solubilisées avec uniquement des macromolécules colloïdales susceptibles de former un trouble et qui peuvent être éliminées lors d'un traitement de purification,
   c) une étape de séparation de la fraction insoluble pour ne conserver que la fraction solubilisée,
   d) une étape de purification de ladite fraction solubilisée contenant les mannoprotéines des parois de cellules de levure, avec au moins une étape permettant l'élimination des macromolécules colloïdales susceptibles de former un trouble et contenues dans la fraction solubilisée,

   l'étape de purification étant suivie d'une concentration finale et éventuellement d'un séchage.

4. Procédé selon la revendication 3, caractérisé en ce que la suspension de parois de cellules de levure est obtenue par l'autolyse d'une crème de levure appartenant de préférence à l'espèce *Saccharomyces cerevisiae* suivie d'une séparation en deux fractions, l'une soluble l'autre insoluble, ladite fraction insoluble correspondant à la suspension de parois de cellules de levure et étant destinée à être mise en oeuvre dans les étapes suivantes du procédé, et notamment dans les étapes d'hydrolyse, de séparation et de purification et ayant les caractéristiques suivantes :

   - teneur en matière sèche comprise entre 5 et 20%, de préférence entre 10 et 15% et encore de préférence entre 12 et 13%,
   - teneur en azote par rapport à la matière sèche comprise entre 1,5 et 3,5%, de préférence entre 1,5 et 3% et encore de préférence entre 1,5 et 2,5%, et encore plus préférentiellement entre 1,5 et 2%,
   - teneur en glucides totaux par rapport à la matière sèche comprise entre 40 et 60%,

- teneur en mannanes par rapport à la teneur en glucides totaux comprise entre 30 et 40%,
- teneur en lipides par rapport à la matière sèche comprise entre 20 et 30%,
- teneur en matières minérales par rapport à la matière sèche comprise entre 5 et 10%,
- teneur en phosphore exprimée en $P_2O_5$ par rapport à la matière sèche comprise entre 2 et 5%, de préférence entre 1,8 et 4%, encore de préférence entre 1,8 et 2,3%.

5. Procédé selon l'une des revendications 3 ou 4, caractérisé en ce que l'étape d'hydrolyse contrôlée des parois de cellules de levure se fait soit par voie enzymatique, soit par voie physico-chimique et est conduite de manière à ce que la fraction solubilisée obtenue après l'étape d'hydrolyse et après l'étape de séparation de la fraction insoluble, ait une valeur de turbidité de moins de 30 NTU, de préférence de moins de 20 NTU, et encore de préférence de moins de 15 NTU et encore plus préférentiellement de moins de 10 NTU lorsqu'elle est mesurée par néphélométrie dans une solution aqueuse diluée contenant 1% de matières sèches, à une valeur de pH comprise entre 5 et 7, de préférence entre 6 et 7.

6. Procédé selon l'une des revendications 3 à 5, caractérisé en ce que l'étape d'hydrolyse contrôlée des parois de cellules de levure se fait à l'aide de la chaleur et est conduite de manière à ce qu'après la séparation de la fraction solubilisée et de la fraction insoluble réalisée au cours de l'étape de séparation, le taux de solubilisation est compris entre 15 et 26% et de préférence entre 18 et 25%, et encore de préférence entre 17 et 20% des matières sèches mises en oeuvre.

7. Procédé selon la revendication 6, caractérisé en ce que l'hydrolyse contrôlée des parois de cellules de levure est menée à une valeur de pH comprise entre 6 et 7, et à une température comprise entre 95 et 100°C, pendant une durée comprise entre 15 et 30 heures, de préférence pendant une durée comprise entre 17 et 25 heures, et encore de préférence entre 17 et 22 heures.

8. Procédé selon l'une des revendications 3 à 7, caractérisé en ce que la suspension de parois de cellules de levure hydrolysés est diluée avec de l'eau jusqu'à contenir entre 1 et 6% de matière sèche, de préférence entre 3 et 5% de matière sèche, à la fin de l'étape d'hydrolyse et avant la séparation de la fraction solubilisée riche en mannoprotéines et de la fraction insoluble.

9. Procédé selon l'une des revendications 3 à 8, caractérisé en ce que la solution contenant les mannoprotéines obtenue après l'étape d'hydrolyse de la suspension de parois de cellules de levure et après l'étape de séparation de la fraction solubilisée et élimination de la fraction insoluble a les caractéristiques suivantes :

- teneur en matière sèche comprise entre 0,5 et 3%, de préférence entre 0,5% et 1,5%,
- teneur en protéines par rapport à la matière sèche comprise entre 15 et 55%, de préférence entre 20 et 50%,
- teneur en glucides totaux par rapport à la matière sèche comprise entre 40 et 80%, de préférence entre 40 et 75%, encore de préférence entre 50 et 67%, lesdits glucides étant composés d'au moins 75% de mannanes, de préférence d'au moins 80% de mannanes, et encore de préférence d'au moins 85% de mannanes,
- teneur en phosphore exprimée en $P_2O_5$ par rapport à la matière sèche comprise entre 1 et 6,00%, de préférence entre 2,75 et 6,00%, et encore de préférence entre 2,75 et 4,5%.

10. Procédé selon l'une des revendications 3 à 9, caractérisé en ce que l'étape de purification des mannoprotéines comprend au moins les étapes suivantes :

a) étape de préconcentration de la fraction solubilisée et séparée jusqu'à une valeur comprise entre 10 et 20% de matière sèche, de préférence entre 13 et 17% ;
b) étape de réfrigération de la fraction préconcentrée et maintien à une température comprise entre 0 et 6°C jusqu'à ce que sa turbidité n'augmente plus ;
c) étape de séparation des macromolécules colloïdales précipitées au froid par clarification centrifuge.

11. Procédé selon la revendication 10, caractérisé par au moins l'une des caractéristiques suivantes :

- le traitement au froid se fait pendant au moins 24 heures,
- la clarification centrifuge est réalisée à au moins 10 000 g sur séparateur auto-débourbeur.

12. Procédé selon l'une des revendications 3 à 10 caractérisé en ce que l'étape de purification des mannoprotéines comprend au moins une microfiltration en profondeur.

**13.** Poudre de mannoprotéines solubles caractérisée en ce qu'elle peut être obtenue par un procédé selon l'une des revendications 3 à 12.

# Figure unique
## PROCEDE d'OBTENTION d'une POUDRE de MANNOPROTEINES SOLUBLES

cellules de levure

extrait de levure ← autolyse — ETAPE 1

séparation crème d'écorces
de levure à 5 à 20 % ES

⟷ hydrolyse contrôlée
enzymatique ou physique — ETAPE 2

écorces hydrolysées

dilution : 1 v crème
pour 1 à 6 v d'eau

écorces hydrolysées diluées
1 à 6 % ES

insolubles (boues)
7 à 9% ES ← séparation centrifuge
- (séparateur à buses) ⟷ ETAPE 3

soluble
0,5 à 3 % ES

⟷ préconcentration sous vide — VARIANTE 1

soluble préconcentré
10 à 20 % ES

soluble préconcentré
10 à 15 % ES

précipitation à froid
des macromolécules colloïdales — ETAPE 4

boues ← débourbage sur clarificateur
centrifuge

soluble préconcentré
et débourbage — VARIANTE 2

VARIANTE 3

microfiltration
sur cartouche
1 μm

microfiltration frontale
profondeur sur plaques
de cellulose

microfiltration
sur cartouche
0,45 μm

concentration sous vide

séchage
(atomisation ou lyophilisation)

MANNOPROTEINES SECHEES

**Office européen des brevets**

## RAPPORT DE RECHERCHE EUROPEENNE

Numéro de la demande

EP 00 40 2916

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 97 05272 A (QUEST INT ;KUNST ANTHONIE (NL); LALOR EOIN (IE); SCHMEDDING DIEDER) 13 février 1997 (1997-02-13) * abrégé * * exemples 1,2 * | 3,13 | C12P21/00 C12H1/14 |
| X | TORABIZADEH H ET AL: "Preparation and characterisation of bioemulsifier from Saccharomyces cerevisiae and its application in food products." LEBENSMITTEL-WISSENSCHAFT UND -TECHNOLOGIE 1996 IRANIAN RES. INST., ORG. FOR SCI. & TECH., 71 FORSAT ST., ENGHELAB AVE., PO BOX 15815-3538, TEHRAN 15819, IRAN, vol. 29, no. 8, pages 734-737, XP000929219 * le document en entier * | 1,2,13 | |
| A | EP 0 790 316 A (QUEST INT) 20 août 1997 (1997-08-20) * le document en entier * | 1-12 | |
| A | WO 96 13571 A (FACULTE D OENOLOGIE ;MOINE VIRGINIE (FR); DUBOURDIEU DENIS (FR)) 9 mai 1996 (1996-05-09) * le document en entier * | 1-12 | **DOMAINES TECHNIQUES RECHERCHES (Int.Cl.7)** C12P C12H |

Le présent rapport a été établi pour toutes les revendications

| Lieu de la recherche | Date d'achèvement de la recherche | Examinateur |
|---|---|---|
| LA HAYE | 16 février 2001 | Lejeune, R |

EPO FORM 1503 03.82 (P04C02)

**EP 1 094 117 A1**

**ANNEXE AU RAPPORT DE RECHERCHE EUROPEENNE
RELATIF A LA DEMANDE DE BREVET EUROPEEN NO.**

EP 00 40 2916

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-02-2001

| Document brevet cité au rapport de recherche | | Date de publication | Membre(s) de la famille de brevet(s) | | Date de publication |
|---|---|---|---|---|---|
| WO 9705272 | A | 13-02-1997 | AU | 6303796 A | 26-02-1997 |
| EP 0790316 | A | 20-08-1997 | AU | 727070 B | 30-11-2000 |
| | | | AU | 1473997 A | 21-08-1997 |
| | | | JP | 10000098 A | 06-01-1998 |
| | | | ZA | 9701271 A | 14-08-1998 |
| WO 9613571 | A | 09-05-1996 | FR | 2726284 A | 03-05-1996 |
| | | | AU | 696819 B | 17-09-1998 |
| | | | AU | 3848395 A | 23-05-1996 |
| | | | BG | 101400 A | 28-11-1997 |
| | | | CA | 2203925 A | 09-05-1996 |
| | | | EP | 0789750 A | 20-08-1997 |
| | | | HU | 77456 A | 28-04-1998 |
| | | | MD | 970226 A | 31-05-1999 |
| | | | NZ | 295194 A | 28-01-1999 |
| | | | US | 6139891 A | 31-10-2000 |

EPO FORM P0460

Pour tout renseignement concernant cette annexe : voir Journal Officiel de l'Office européen des brevets, No.12/82